# EUROPEAN PATENT SPECIFICATION

(11) **EP 1 351 669 B1**
(45) Date of publication and mention of the grant of the patent: **20.10.2004**
(21) Application number: 01999369.0
(22) Date of filing: 05.12.2001
(51) Int. Cl.: A61K 31/05

(54) **CLEAR PROPOFOL COMPOSITIONS**
KLARE PROPOFOLZUSAMMENSETZUNGEN
COMPOSITIONS INCOLORES ET STABLES A BASE DE PROPOFOL

(30) Priority: 07.12.2000 IN DE110600
(43) Date of publication of application: 15.10.2003
(73) Proprietor: Bharat Serums & Vaccines Ltd., Wagle Estate, Thane 400 604 (IN)
(72) Inventor: PAI, Srikanth Annappa, Wagle Estate 400 604 Thane (IN); RIVANKAR, Sangeeta Hanurmesh, Wagle Estate 400 604 Thane (IN); KOCHAREKAR, Shilpa Sudhakar 204/A, Rachna Niraj, School Kopri 400603 Thane (West) (IN)
(74) Representative: Casey, Lindsay Joseph
(86) International application number: PCT/IN2001/000213
(87) International publication number: WO 2002/045709

(56) References cited:
- WO-A-00/44369
- WO-A-00/78301
- WO-A-01/97796
- WO-A-98/30205
- WO-A-99/39696
- US-A- 5 714 520

## Description

This invention relates to a process for preparation of a clear, sterile anaesthetic composition of Propofol suitable for parenteral administration

### Background of the Invention :

Propofol (2, 6-diisopropyl phenol) is an intravenous anesthetic agent characterised by a short recovery time. It has the desirable property of rapid onset and offset of the anaesthetic effect following intravenous administration and minimal accumulation on long-term administration.

Propofol even though is a preferred anesthetic agent, has posed a big challenge to the formulators since its invention because of its aqueous insolubility. It was at first formulated as a 1% aqueous solution containing nonionic surfactant Cremophor EL as a solubiliser. However Cremophor EL has been implicated in some adverse reactions when administered intravenously, including anaphylactoid reactions. Subsequently, the anesthetic agent was formulated as an oil-in-water emulsion containing 1% w/v Propofol with 10% w/v soybean oil and 1.2% w/v purified egg phosphatide.

Lipid based emulsions, however, suffer from several limitations which are as follows.
- Improper storage results in poor physical stability.
- On storage produces free fatty acid which is toxic.
- It has potential to cause embolism because of higher oil globule size.
- Causes pain on injection (contains oil in the formulation).
- During administration online microbial filter cannot be used.
- Cannot be visually inspected for foreign particles before administration as the product has milky appearance.
- Can be selectively admixed with only a few injectable products before administration.
- During administration, strict aseptic techniques must be followed as emulsion system is highly susceptible for bacterial growth.
- During maintenance anaesthesia, infusion tubes require to be changed frequently as the product inside infusion tube may support bacterial growth.
- Contains Egg lecithin as an emulsifier. Being from an animal source, may produce allergy in some patients.
- Increases concentration of plasma phospholipid, decreases clearance of triglycerides causing hypertriglyceridemia, hypercholesterolemia.
- Before autoclaving, the product cannot be filtered through 0.22µ filter.

The inherent limitations of the emulsion formulation as mentioned above could be overcome by eliminating vegetable oil and phospholipids from the formulation. Development of a composition of this type of Propofol formulation (removing vegetable oil and phospholipids) for parenteral administration is possible if Propofol is made soluble in aqueous phase using a solubiliser, as Propofol is an oily liquid, insoluble in water.

WO00/78301 describes an anaesthetic composition for intravenous injection containing Propofol and Poloxamer as the surfactant. Optionally it can contain at least one more surfactant selected from the group consisting of Solutol HS15, Egg lecithin, Labrasol, Polyoxy-10-oleyl ether, Tween, Ethanol and Polyethylene glycol. The composition is prepared in the form of a microemulsion having particle size below 100nm and can be aseptically filtered.

WO01/64187 also describes an similar anaesthetic composition for intravenous injection containing Propofol and Poloxamer. This invention defines the type of polymers that can be used i.e. Propylene oxide portion is at least 2000D while the Ethylene oxide portion is at least 40% w/w. Under formulation stability, the influence of pH and added electrolyte (sodium chloride) on the hydrodynamic radii of micelles has been discussed. However, the stability of the active ingredient Propofol has not been considered in above two patents Poloxamer has been reported to be incompatible with phenols. (Ref. Martindale 32^{nd} edition, Pg. 1326) and hence it is doubtful that such compositions would retain propofol activity for a long time.

The main object of the present invention is to develop a clear, stable sterile aqueous composition of Propofol overcoming drawbacks of the existing formulation and also the drawbacks of the prior art.

### Summary of the Invention :

Accordingly the present invention relates to a clear stable anaesthetic composition suitable for parenteral administration comprising Propofol (1mg/ml to 20mg/ml of the composition), d-Alpha Tocopheryl Polyethylene Glycol 1000 Succinate (TPGS) keeping the ratio of propofol to TPGS at least 1: 10 (by wt.) and the content of TPGS from 1 to 20 % w/v, in the composition, and water with or without parenterally acceptable conventional additives.

The process of manufacturing the anaesthetic composition of present invention comprises
a) dissolving TPGS in water to give TPGS solution;
b) adding propofol under mixing to said TPGS solution to give anaesthetic composition;
c) said additives if required may be added to water, TPGS solution or anaesthetic composition formed;
d) making up the volume with water to the desired level of propofol in the anaesthetic composition;
e) filtering the composition obtained at the end of step (d) through 2µ and 0.2µ filter;
f) filling the filtrate obtained at the end of step (e) in containers such as vials, ampoules, plastic containers followed by nitrogen purging and sealing the filled containers;
g) autoclaving the sealed containers filled with said filtrate.

### Detailed description of embodiments of the Invention :

While developing an anaesthetic composition of Propofol in light of the object set as above, we found that some hydrophobic therapeutic drugs are dissolved in α-tocopherol, an oily material and then the α-Tocopherol solution is emulsified using TPGS as emulsifier (Ref: W098/30205). As we intended to avoid oily composition, after lot of experimentation we found that by using sufficiently large quantities of TPGS we can solubilise the anaesthetic hydrophobic propofol in water giving a composition suitable for parenteral administration.

WO98/30205 discloses substantially ethanol-free self-emulsifying drug delivery system. The pharmaceutical compositions of the invention are typically formed by dissolving a therapeutic agent in ethanol to form a therapeutic agent solution. Alpha-tocopherol is then added to the therapeutic agent solution to form an alpha-tocopherol and therapeutic agent solution. Next, the ethanol is removed to a form a substantially ethanol-free alpha-tocopherol and therapeutic agent solution. The substantially ethanol free alpha tocopherol and therapeutic agent solution is blended with and without an aqueous phase incorporating a surfactant to form a pre-emulsion. For intravenous delivery the pre-emulsion is then homogenised to form a fine emulsion.

In this delivery system ethanol is used to dissolve the therapeutic agent and incorporated into large amount of α-tocopherol which is essential for holding the drug in solution.

TPGS is a water soluble form of alpha-tocopherol prepared by esterifying the acid group of crystalline d-alpha-tocopheryl acid succinate with polyethylene glycol 1000. TPGS is very stable and does not hydrolyze under normal conditions.

TPGS melts at 41°C and degrades at temperatures above 199°C. TPGS has a solubility of 20g% in water at 20°C. TPGS has an HLB value in the region of 15 - 19.

For dissolving TPGS in water, TPGS is required to be added to hot water. The preferred temperature of water could be from 45°C to 100°C.

TPGS does not degrade if exposed to oxygen, heat, light or oxidising agents. It is unstable to alkali.

While preparing the composition of our present invention, after lot of experimentation we found that an oil soluble anaesthetic agent Propofol can be solubilised in water with the help of TPGS, without using any tocopherol or any oily phase if we use higher amounts of TPGS to give a solubilised product containing Propofol

The composition of present invention gives a stable product when stored under controlled conditions of temperature i.e. under refrigeration.

The Propofol content of the composition of present invention is from 1mg/ml to 20mg/ml, preferably from 2mg/ml to 20mg/ml, more preferably 10mg/ml.

The TPGS content of the composition of present invention is from 10mg/ml to 200mg/ml, preferably from 100mg/ml to 150mg/ml, more preferably 100mg/ml.

The present invention gives a clear stable, sterile aqueous composition suitable for parenteral administration without addition of any conventional additives.

However, in another embodiment of the invention addition of conventional additives if required by parenteral dosage form may be added to the aqueous solution at any stage of preparation.

Parenterally acceptable conventional additives when added are selected from a group of additives such as buffers, tonicity modifying agents, preservatives, antioxidants at conventional usage levels.

Any of the parenterally used buffer can be used in the composition of present invention which is selected from a group of buffers such as phosphate buffer, glycine buffer, citrate buffer or a mixture thereof. Phosphate buffer is preferred whenever buffer is used. Phosphate buffers with different compositions of sodium dihydrogen phosphate, disodium hydrogen phosphate, phosphoric acid, sodium hydroxide can be used to give a desired pH to the composition. A pH range of 4.0 - 8.0 of the final composition is preferred.

The composition of present invention can also contain tonicity modifying agent to make the composition isotonic with the blood. The tonicity modifying agent is selected from a group of partenterally acceptable compounds such as dextrose, sodium chloride, mannitol, sorbitol, glycerin, propylene glycol or a mixture thereof Glycerin is particularly preferred as tonicity modifying agent at a concentration of 2 - 3% w/v of the composition when used. 2.25% w/v of the glycerin is more preferred in the final composition.

Preservatives when used in the composition of present invention are selected from a group of parenterally acceptable compounds such as disodium edetate, benzyl alcohol, sodium benzoate or a mixture thereof. Disodium edetate is the preferred preservative in a concentration range of 0.0025% to 0.01% w/v of the composition when used.

The composition of present invention can also contain antioxidants which are selected from a group of parenterally acceptable compounds such as ascorbyl-6 palmitate, ascorbic acid, salts of ascorbic acid. One of the advantages of TPGS is that it by itself acts as an antioxidising agent and additional antioxidants may not be necessary.

It is to be understood that the invention is not limited to the disclosed embodiments, but may be practised within the full scope of claims appended herewith.

### Examples :

The invention will now be illustrated by way of examples. The examples are by way of illustration only.

All the raw materials used in this example were of parenteral grade. Equipments used were of conventional nature. Entire processing was done in an area with a controlled environment. Nitrogen cover was provided while processing the batch.

The following materials were used in the Examples
a) Propofol was procured from Cilag AG
b) TPGS complying with United States National Formulary (USNF) specification was procured from M/s. Eastman Chemical Ltd.
c) Water complying with "Water for Injection" specifications of Indian Pharmacopoeia was used.
d) Glycerin complying with specifications of Indian Pharmacopoeia was used.
e) Disodium edetate complying with specifications of Indian Pharmacopoeia was used.
f) Potassium dihydrogen phosphate complying with specifications of USNF was used.
g) Disodium hydrogen phosphate complying with specifications of Indian Pharmacopoeia was used.
h) Diprivan - Propofol Emulsion (10mg/ml) available commercially was used.

### Example I :

TPGS was melted and 20g of molten TPGS was added to boiling water (170ml). Stirred till TPGS got dissolved completely. Propofol (2g) was added to TPGS solution under stirring. The product was stirred till a clear solution was obtained. Volume was made upto 200ml with water. Filtration was carried out using 2µ prefilter and 0.22µ membrane filter. The product was filled into sterile pyrogen free glass vials under nitrogen cover under laminar flow. The vials were closed using flurotec rubber stoppers and sealed using aluminium seals. The filled and sealed vials were autoclaved at 121°C for 20 minutes.

| **STABILITY DATA OF PRODUCT OF EXAMPLE I UNDER RECOMMENDED STORAGE CONDITIONS of 2 - 8°C** | | |
|---|---|---|
| **PERIOD** | **APPEARANCE** | **PROPOFOL CONTENT** |
| Initial | Clear slightly yellowish liquid | 101.63% |
| 6 Months | Clear slightly yellowish liquid | 100.42% |
| 9 Months | Clear slightly yellowish liquid | 99.47% |

The above stability data indicates that the product prepared in Example I is suitable for commercial marketing.

### Example II :

Example II was same as Example I except that Glycerin (4.5gms) was added after solubilising Propofol.

### Example III :

Example III was same as Example II except that Disodium Edetate (0.011g) was dissolved in boiling water before addition of TPGS.

### Example IV :

Example IV was same as Example I except that Phosphate buffer of pH 5.5 was used in place of water.

Phosphate buffer of pH 5.5 was prepared by mixing 96.4ml of "Solution I" with 3.6ml of "Solution II".
1) **Solution I** - Dissolve 13.61g of Potassium dihydrogen phosphate in sufficient water to produce 1000ml.
2) **Solution II** - Dissolve 35.81g of Disodium hydrogen phosphate in sufficient water to produce 1000ml.

### Example V

Same as Example I except that 16gms of TPGS was used instead of 20g. The product obtained was hazy indicating incomplete solubilisation of propofol.

### Example VI

Propofol composition prepared in Example I was subjected to in-vivo toxicity studies in mice.

### Single dose toxicity study in mice

### Material and method

***Test System*** : Female Swiss albino mice in the weight range of 20-22 g were obtained from the animal house of Bharat Serums & Vaccines Ltd (BSVL) and employed for the study. The animals were provided with standard chow and Aquaguard™ water, *ad libitum.*

***Test Material*** : Propofol composition (10 mg/ml) prepared in Example I, was administered intravenously.

***Comparative material* :** Diprivan - Propofol emulsion (10mg/ml) available commercially was administered intravenously.

All animals (8 from each group) were observed for signs of clinical toxicity if any and for mortality for a period of 72 hours. The percent mortality was calculated for all the doses. The results are given in the following table.

| **Dose (mg/kg body wt.)** | **% mortality in sample of Example I** | % **mortality in Diprivan sample** |
|---|---|---|
| 35 | 0 | 0 |
| 40 | 25 | 25 |
| 45 | 37.5 | 37.5 |
| 50 | 50 | 50 |

### Observations :

The groups which received Propofol composition from Example I showed similar signs of toxicity as compared to the group which received Diprivan.

### Example VII

Propofol composition prepared in Example I was subjected to efficacy study in mice.

### Material and method

***Test System*** : Female Swiss albino mice in the weight range of 20-22 g were obtained from the animal house of Bharat Serums & Vaccines Ltd (BSVL) and employed for the study. The animals were provided with standard chow and Aquaguard™ water, *ad libitum.*

***Test Material*** : Propofol composition (10 mg/ml) prepared in Example I, was administered intravenously at a dose of 35mg/kg.

***Comparative material*** : Diprivan - Propofol emulsion (10mg/ml) available commercially was administered intravenously at a dose of 35mg/kg.

All the animals (8 from each group) were observed for the time taken to go into anaesthesia and the time taken to come out of anaesthesia. The observations are as follows :

| **AVERAGE INDUCTION TIME** | | **AVERAGE RECOVERY TIME** | |
|---|---|---|---|
| **Example I** | **Diprivan** | **Example I** | **Diprivan** |
| 4.2 sec. | 4.1 sec. | 8.6 min. | 7.9 min. |

The stability studies, toxicity studies and efficacy studies on Example I demonstrated that the composition of present invention is stable overcoming all the disadvantages of the emulsion formulation discussed earlier and comparable to Diprivan in toxicity and efficacy.

### Advantages of the present invention :

The present invention gives a clear sterile anaesthetic composition that overcomes the disadvantages of emulsion formulation discussed earlier and gives a composition which has many advantages some of which are as follows:
i. The composition is clear, can be visually inspected before administration and can be administered with the use of on-line microbial filter.
ii. The composition does not contain phospholipids. Hence plasma phospholipids are unaffected on parenteral administration of the composition.
iii. The composition does not cause any change in triglyceride clearance
iv. The composition can be mixed with any of the commonly used diluents before administration.

## Claims

1. A clear stable anaesthetic composition suitable for parenteral administration comprising Propofol (1mg/ml to 20mg/ml of the composition), d-Alpha Tocopheryl Polyethylene Glycol 1000 Succinate (TPGS) keeping the ratio of propofol to TPGS at least 1: 10 (by wt.) and the content of TPGS from 1 to 20 % w/v in the composition, and water; with or without parenterally acceptable additives.

2. A clear stable anaesthetic composition suitable for parenteral administration as claimed in claim 1 wherein the content of Propofol is about 10mg/ml of the composition.

3. A clear stable anaesthetic composition suitable for parenteral administration as claimed in any of claims 1 or 2 wherein the content of TPGS is from 100mg/ml to 150mg/ml of the composition

4. A clear stable anaesthetic composition suitable for parenteral administration as claimed in any of claims 1 to 3 wherein the parenterally acceptable additives are selected from group of additives such as buffers, tonicity modifying agents, preservatives, antioxidants.

5. A clear stable anaesthetic composition suitable for parenteral administration as claimed in any of claims 1 to 4 wherein the buffer used is selected from a group of parenterally acceptable buffers such as phosphate buffer, glycine buffer, citrate buffer or a mixture thereof.

6. A clear stable anaesthetic composition suitable for parenteral administration as claimed in any of claims 1 to 5 wherein the tonicity modifying agent used is selected from a group of parenterally acceptable compounds such as dextrose, sodium chloride, mannitol, sorbitol, glycerin, propylene glycol or a mixture thereof.

7. A clear stable anaesthetic composition suitable for parenteral administration as claimed in any of claims 1 to 6 wherein the tonicity modifying agent used is glycerin.

8. A clear stable anaesthetic composition suitable for parenteral administration as claimed in any of claims 1 to 7 wherein the tonicity modifying agent used is propylene glycoL

9. A clear stable anaesthetic composition suitable for parenteral administration as claimed in any of claims 1 to 8 wherein the preservative used is selected from a group of parenterally acceptable compounds such as disodium edetate, benzyl alcohol, sodium benzoate or a mixture thereof.

10. A process for preparation of clear stable anaesthetic composition suitable for parenteral administration as claimed in any of claims 1 to 9, comprising
a) dissolving TPGS in water to give TPGS solution;
b) adding propofol under mixing to said TPGS solution to give anaesthetic composition;
c) said additives if required may be added to water, TPGS solution or anaesthetic composition formed;
d) making up the volume with water to the desired level of propofol in the anaesthetic composition;
e) filtering the composition obtained at the end of step (d) through 2µ and 0.2µ filter;
f) filling the filtrate obtained at the end of step (e) in containers such as vials, ampoules, plastic containers followed by nitrogen purging and sealing the filled containers;
g) autoclaving the sealed containers filled with said filtrate.

11. A clear stable anaesthetic composition suitable for parenteral administration as claimed in any of claims 1-9 prepared by the process as claimed in claim 10.

## Patentansprüche

1. Klare, stabile Anästhetikum-Zusammensetzung, die zur parenteralen Verabreichung geeignet ist, umfassend Propofol (1 mg/ml bis 20 mg/ml der Zusammensetzung), D-α-Tocopheryl-Polyethylenglycol-1000-Succinat (TPGS), wobei das Verhältnis von Propofol zu TPGS bei mindestens 1 : 10 (Gewichtsverhältnis) und der TPGS-Gehalt von 1 bis 20 % (w/v) in der Zusammensetzung aufrechterhalten wird und Wasser; mit oder ohne parenteral verträglichen Zusatzstoffen.

2. Klare, stabile Anästhetikum-Zusammensetzung, die zur parenteralen Verabreichung nach Anspruch 1 geeignet ist, worin der Propofol-Gehalt ca. 10 mg/ml der Zusammensetzung beträgt.

3. Klare, stabile Anästhetikum-Zusammensetzung, die zur parenteralen Verabreichung nach einem der Ansprüche 1 oder 2 geeignet ist, worin der TPGS-Gehalt von 100 mg/ml bis 150 mg/ml der Zusammensetzung beträgt.

4. Klare, stabile Anästhetikum-Zusammensetzung, die zur parenteralen Verabreichung nach einem der Ansprüche 1 bis 3 geeignet ist, worin die parenteral verträglichen Zusatzstoffe aus der Gruppe von Zusatzstoffen, wie zum Beispiel Puffern, Tonizitätsmodifikationsmitteln, Konservierungsmitteln und Antioxidanzien ausgewählt sind.

5. Klare, stabile Anästhetikum-Zusammensetzung, die zur parenteralen Verabreichung nach einem der Ansprüche 1 bis 4 geeignet ist, worin der verwendete Puffer aus einer Gruppe von parenteral verträglichen Puffern, wie zum Beispiel Phosphatpuffer, Glycinpuffer, Citratpuffer oder einem Gemisch davon ausgewählt ist.

6. Klare, stabile Anästhetikum-Zusammensetzung, die zur parenteralen Verabreichung nach einem der Ansprüche 1 bis 5 geeignet ist, worin das verwendete Tonizitätsmodifikationsmittel aus einer Gruppe von parenteral verträglichen Verbindungen, wie zum Beispiel Dextrose, Natriumchlorid, Mannitol, Sorbitol, Glycerin, Propylenglycol oder einem Gemisch davon ausgewählt ist.

7. Klare, stabile Anästhetikum-Zusammensetzung, die zur parenteralen Verabreichung nach einem der Ansprüche 1 bis 6 geeignet ist, worin das verwendete Tonizitätsmodifikationsmittel Glycerin ist.

8. Klare, stabile Anästhetikum-Zusammensetzung, die zur parenteralen Verabreichung nach einem der Ansprüche 1 bis 7 geeignet ist, worin das verwendete Tonizitätsmodifikationsmittel Propylenglycol ist.

9. Klare, stabile Anästhetikum-Zusammensetzung, die zur parenteralen Verabreichung nach einem der Ansprüche 1 bis 8 geeignet ist, worin das verwendete Konservierungsmittel aus einer Gruppe von parenteral verträglichen Verbindungen, wie zum Beispiel Dinatriumedetat, Benzylalkohol, Natriumbenzoat oder einem Gemisch davon ausgewählt ist.

10. Verfahren zur Herstellung einer klaren, stabilen Anästhetikum-Zusammensetzung, die zur parenteralen Verabreichung nach einem der Ansprüche 1 bis 9 geeignet ist, umfassend
a) Auflösen von TPGS in Wasser, um eine TPGS-Lösung zu geben;
b) Zufügen von Propofol unter Beimischung zu genannter TPGS-Lösung, um eine Anästhetikum-Zusammensetzung zu geben;
c) genannte Zusatzstoffe, die gegebenenfalls Wasser, einer TPGS-Lösung oder einer gebildeten Anästhetikum-Zusammensetzung zugefügt werden können;
d) Auffüllen des Volumens mit Wasser auf den gewünschten Propofol-Spiegel in der Anästhetikum-Zusammensetzung;
e) Filtrieren der am Ende von Schritt (d) erhaltenen Zusammensetzung durch ein 2-µ- und 0,2-µ-Filter;
f) Abfüllen des am Ende von Schritt (e) erhaltenen Filtrats in Behältnisse, wie zum Beispiel Durchstichflaschen, Ampullen und Kunststoffbehälter, gefolgt durch Spülen mit Stickstoff und festem Verschließen der gefüllten Behältnisse;
g) Autoklavieren der mit genanntem Filtrat gefüllten, fest verschlossenen Behältnisse.

11. Klare, stabile Anästhetikum-Zusammensetzung, die zur parenteralen Verabreichung nach einem der Ansprüche 1 - 9 geeignet ist, die durch das Verfahren nach Anspruch 10 hergestellt ist.

## Revendications

1. Composition anesthésique transparente et stable adaptée à l'administration parentérale comprenant du propofol (1 mg/ml à 20 mg/ml de la composition), du tocophéryl polyéthylène glycol 1000 succinate (TPGS) d-alpha, dont le rapport poids / poids du propofol à TPGS est maintenu à au moins 1 / 10, et dont la teneur en TPGS est de 1 à 20 % p/v, et de l'eau, avec ou sans additifs parentéralement acceptables.

2. Composition anesthésique transparente et stable adaptée à l'administration parentérale selon la revendication 1, dont la teneur en propofol est de l'ordre de 10 mg/ml.

3. Composition anesthésique transparente et stable adaptée à l'administration parentérale selon la revendication 1 ou la revendication 2, dont la teneur en TPGS est de 100 mg/ml à 150 mg/ml.

4. Composition anesthésique transparente et stable adaptée à l'administration parentérale selon l'une quelconque des revendications 1 à 3, dans laquelle les additifs parentéralement acceptables sont sélectionnés parmi un groupe d'additifs tels que les tampons, les agents modifiant la tonicité, les agents de conservation et les antioxydants.

5. Composition anesthésique transparente et stable adaptée à l'administration parentérale selon l'une quelconque des revendications 1 à 4, dans laquelle le tampon utilisé est sélectionné parmi un groupe de tampons parentéralement acceptables tels que le tampon de phosphate, le tampon de glycine, le tampon de citrate ou un mélange de ceux-ci.

6. Composition anesthésique transparente et stable adaptée à l'administration parentérale selon l'une quelconque des revendications 1 à 5, dans laquelle l'agent modifiant la tonicité utilisé est sélectionné parmi un groupe de composés parentéralement acceptables tels que le dextrose, le chlorure de sodium, le mannitol, le sorbitol, la glycérine, le propylène glycol ou un mélange de ceux-ci.

7. Composition anesthésique transparente et stable adaptée à l'administration parentérale selon l'une quelconque des revendications 1 à 6, dans laquelle l'agent modifiant la tonicité utilisé est la glycérine.

8. Composition anesthésique transparente et stable adaptée à l'administration parentérale selon l'une quelconque des revendications 1 à 7, dans laquelle l'agent modifiant la tonicité utilisé est le propylène glycol.

9. Composition anesthésique transparente et stable adaptée à l'administration parentérale selon l'une quelconque des revendications 1 à 8, dans laquelle l'agent de conservation utilisé est sélectionné parmi un groupe de composés parentéralement acceptables tels que l'acide édétique disodique, l'alcool benzylique, le benzoate de sodium ou un mélange de ceux-ci.

10. Procédé pour la préparation d'une composition anesthésique transparente et stable adaptée à l'administration parentérale selon l'une quelconque des revendications 1 à 9, comprenant les étapes suivantes :
a) la dissolution du TPGS dans de l'eau pour obtenir une solution de TPGS ;
b) l'addition du propofol à ladite solution de TPGS sous agitation, de manière à obtenir la composition anesthésique ;
c) si besoin est, l'addition desdits additifs à l'eau, à la solution de TPGS ou à la composition anesthésique formée ;
d) l'ajustement au volume requis avec de l'eau jusqu'à la concentration de propofol désirée dans ladite composition ;
e) le filtrage de ladite composition obtenue à la fin de l'étape (d) à travers un filtre de 2 µ et de 0,2 µ;
f) le déversement dudit filtrat obtenu à la fin de l'étape (e) dans des récipients tels que des flacons, des ampoules ou des récipients en plastique, puis purge à l'azote et scellage des récipients remplis ;
g) le traitement des récipients scellés remplis avec ledit filtrat à l'autoclave.

11. Composition anesthésique transparente et stable adaptée à l'administration parentérale selon l'une quelconque des revendications 1 à 9, préparée suivant le procédé selon la revendication 10.
